# EUROPEAN PATENT APPLICATION

(11) **EP 3 133 378 A1**
(43) Date of publication of application: **22.02.2017**
(21) Application number: 16184904.7
(22) Date of filing: 19.08.2016
(51) Int. Cl.: G01G 19/44, G01G 21/28, G01G 19/50

(54) **WEIGHING SCALE**

(30) Priority: 19.08.2015 US 201562206912 P; 19.08.2015 US 201562206923 P
(71) Applicant: ScaleThings Sp. z o.o., 00-195 Warsaw (PL)
(72) Inventor: AVRAMOVICH, Eyal, 4520029 Hod Hasharon (IL)
(74) Representative: Algemeen Octrooi- en Merkenbureau B.V.

(57) **Abstract**

A Weighing scale which includes a platform, electronics housing and a rotating member. The platform includes a plurality of load cells. The electronic housing includes a processor coupled with the load cells for determining the weight of a load when the load is located on the platform. The rotating member rotatebly couples the platform with the electronics housing such that the electronic housing and the platform can rotate one with respect to the other.

## Description

### FIELD OF THE DISCLOSED TECHNIQUE

The disclosed technique relates to weighing scales in general, and to weighing scales with reduced thickness, in particular.

### BACKGROUND OF THE DISCLOSED TECHNIQUE

A majority of weight scales today exhibit a thickness above 20mm. This thickness may become inconvenient and present a potentially harmful obstacle near hallways especially for the elderly or the very young and for all people during dark periods (e.g., at night).
European Patent Application EP20080171466, to Oseko et al, entitled "Electronic Weighing Scale" directs to weight scale comprising six or more load cell sensors embedded inside mounting holes in the scaling glass. The control and display components of the scale are also embedded in an embedding hole in the scaling glass. The glass has a top cover and the bottom has a supportive base.
Chinese Utility Model publication to CN201683663 to Shiquan Yu, entitled "Weighing Door Mat" directs to a door mat overlaid on top of a weighing scale. Thus, a person can weigh herself or himself while entering or exiting their home.

### SUMMARY OF THE PRESENT DISCLOSED TECHNIQUE

It is an object of the disclosed technique to provide a novel weighing scales. In accordance with the disclosed technique, there is thus provided weighing scales which includes a platform, electronics housing and a rotating member. The platform includes a plurality of load cells. The electronic housing includes a processor coupled with the load cells for determining the weight of a load when the load is located on the platform. The rotating member rotatebly couples the platform with the electronics housing such that the electronic housing and the platform can rotate one with respect to the other.

### BRIEF DESCRIPTION OF THE DRAWINGS

The disclosed technique will be understood and appreciated more fully from the following detailed description taken in conjunction with the drawings in which:
Figures 1A-1D are a schematic illustration of weighing scales, constructed and operative in accordance with an embodiment of the disclosed technique; and
Figures 2A-2C are schematic illustration of scales constructed and operative in accordance with another embodiment of the disclosed technique.

### DETAILED DESCRIPTION OF THE EMBODIMENTS

The disclosed technique overcomes the disadvantages of the prior art by providing weighing scales which includes a platform and an electronics housing rotatebly coupled therebetween. When a load is placed on the platform, the distance between the platform and the floor may be reduced. However, since the platform and electronic housing 104 are rotatebly coupled therebetween the platform may move up or down independently from electronics housing without affecting the weight measurement.

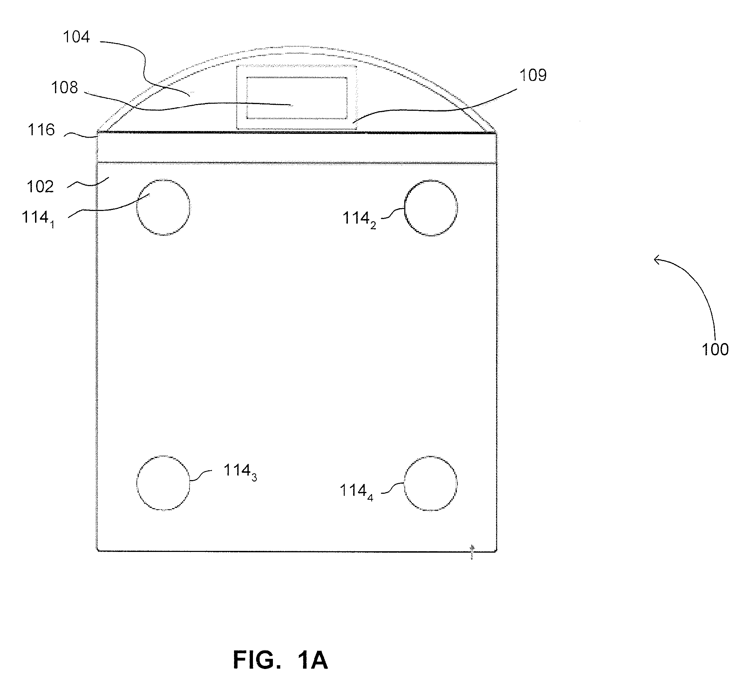

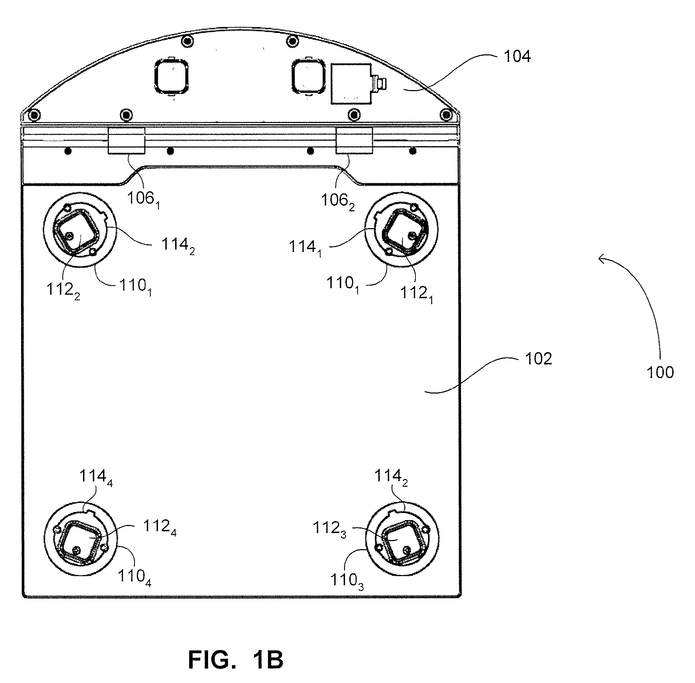

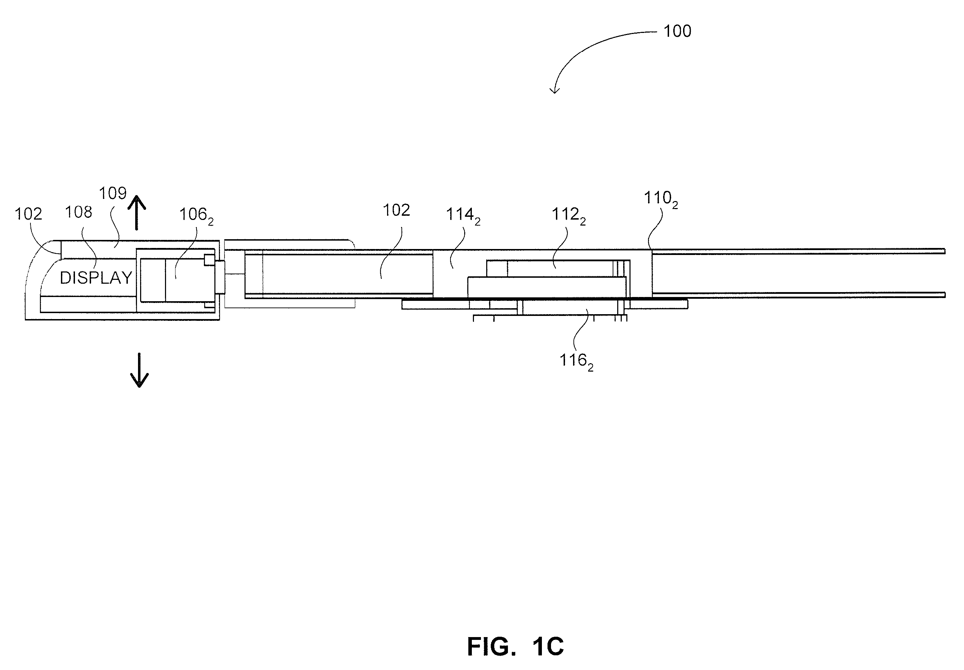

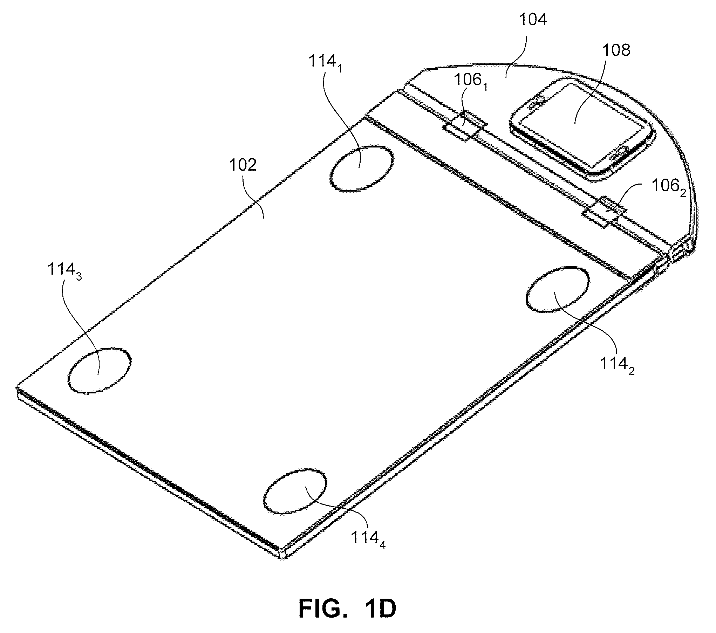

Reference is now made to Figures 1A-1D, which are a schematic illustration of weighing scales, generally referenced 100, constructed and operative in accordance with an embodiment of the disclosed technique. Figure 1A depicts a top view of scales 100, Figure 1B depicts a bottom view of scales 100, Figure 1C depicts a fragmental side view of scales 100 and Figure 1D depicts an isometric view of scales 100. Scales 100 include a platform 102 (e.g., made out of glass, carbon fiber or metal), electronics housing 104. Platform 102 is rotatebly coupled with electronics housing 104 via a rotating member or members such as hinges 106₁ and 106₂, such that electronic housing 104 and platform 102 can rotate one with respect to the other. Alternatively, the rotating member may be made of a flexible material (e.g., rubber or silicon) coupled with both platform 102 and electronics housing 104. Electronics housing 104 includes a display 108 as well as additional components such as a processor and power supply. Display 108 may be covered with glass cover 109 to prevent damage thereto when a load is accidently placed thereon.

Platform 102 includes perforations 110₁, 110₂, 110₃ and 110₄ in which respective load cells 112₁, 112₂, 112₃ and 112₄ are located. Perforations 110₁, 110₂, 110₃ and 110₄ may extend either completely or partially through platform 102. Optionally load cells 112₁, 112₂, 112₃ and 112₄ are mounted on respective cell housings 114₁, 114₂, 114₃ and 114₄ which are located in perforations 110₁, 110₂, 110₃ and 110₄. Cell housings 114₁, 114₂, 114₃ and 114₄ are made, for example, from metal (e.g., aluminum, nickel and the like) and are covered by cell covers, such as cell cover 116₂ (e.g., made of plastic, rubber, silicon and the like), which protect the respective one of load cells 112₁, 112₂, 112₃ and 112₄ in cell housings 114₁, 114₂, 114₃ and 114₄. Cell covers also protect the floor from damage and prevent motion of scales 100 when a load is placed on platform 102. The cell covers protrude from the bottom of platform 102 and are in contact with the respective load cells 112₁, 112₂, 112₃ and 112₄. Load cells 112₁, 112₂, 112₃ and 112₄ are coupled with the processor within electronic housing 104. The processor is further coupled with display 106.

When scales 100 is placed, for example, on the floor and a load is placed on platform 102, load cells 112₁, 112₂, 112₃ and 112₄ are pressed between platform 102 and the floor and specifically between the upper part of cell housings 114₁, 114₂, 114₃ and 114₄ and the cell covers thereof. The force applied on load cells 112₁, 112₂, 112₃ and 112₄ by the load deforms load cells 112₁, 112₂, 112₃ and 112₄ which produce a respective signal corresponding to the force applied thereon. The electric signals from load cells 112₁, 112₂, 112₃ and 112₄ are provided to the processor which determines the weight of the load and displays this weight on display 108. As the load is placed on platform 102, the distance between platform 102 and the floor may be reduced. However, since platform 102 and electronic housing 104 are rotatebly coupled therebetween by a rotating member or members such as hinges 106₁ and 106₂, platform 102 may move up or down independently from electronics housing 104 without affecting the weight measurement. Furthermore, separating display 108 from platform 102 saves cutting an additional hole in platform 102 for display 108, which results in platform 102 being less fragile. This enables employing a thinner platform.

It is noted that scales 100 is not limited to measuring weight scale but may include additional sensors such as fat sensors and modules such as a Body Mass Index (BMI) sensor, a wireless data transceiver or an alarm clock alarm. The wireless data transceiver transmits the information measured by the sensors in scales 100 (i.e., either the weight of the load, the BMI or the fat or any combination thereof), for example, to portable devices such as cellphones or tablet computers or to a database for storage. The alarm clock may sound an alarm at a designated time of the day and may, for example, stops the alarm only after a load is placed on scales 100, (e.g., a user is standing up from his bed and applies hers or his weight on the weight sensors).

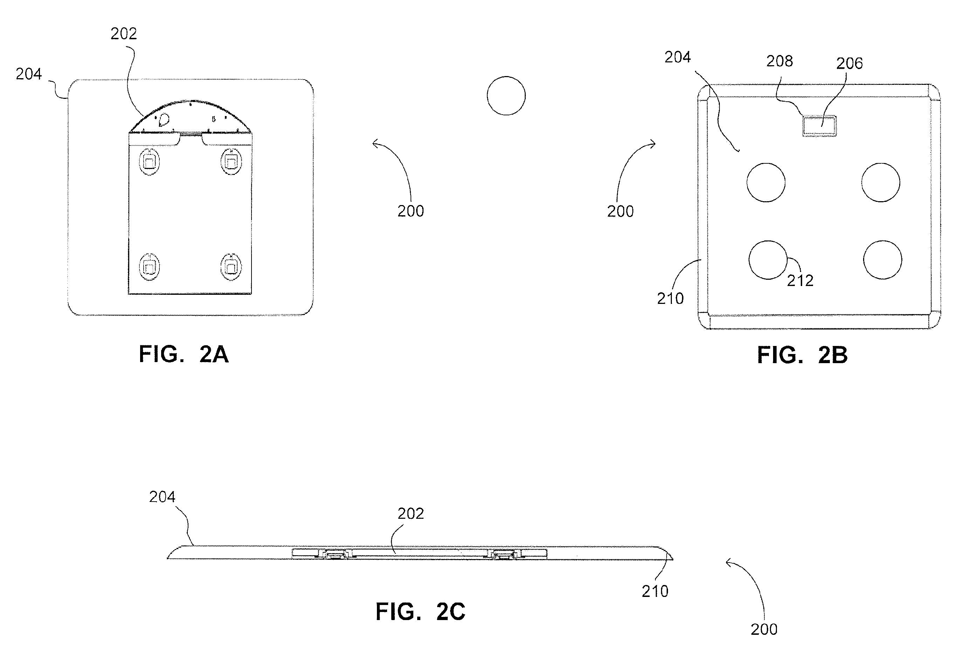

The thinner platform enables covering scales according to the disclosed technique with a rug for aesthetic, comfort and safety purposes. Reference is now made to Figures 2A-2C, which are schematic illustration of scales, generally referenced 200, constructed and operative in accordance with another embodiment of the disclosed technique. Figure 2A depicts a bottom view of scales 200, Figure 1B depicts a top view of scales 200 and Figure 2C depicts a side view of scales 200. Scales 200 include scales 202 covered with a rug 204. Scales 202 are similar to scale 100 described above in conjunction with Figures 1A-1D. Rug 204 includes an opening 208 for display 206 of scales 202. The edges 210 0f rug 208 exhibit a declination toward the floor, which transforms the step onto scale 200 into a smooth ramp, which reduces the risk of the scale edges becoming an inconvenient and harmful obstacle on the floor (e.g., in hallways, bathrooms and the like). Rag 204 optionally included additional openings such as opening 212 enabling contact with BMI sensors on scale 202. Alternatively, the BMI sensor are placed on rug 204 itself. Furthermore, the material employed within the rug and around the scales is a hard foam (e.g., EVA). Thus the user experiences one even level with a similar level of hardness while stepping on the rug and the scales rather than two different levels of hardness (i.e., one of the scales and one of the rug).

It will be appreciated by persons skilled in the art that the disclosed technique is not limited to what has been particularly shown and described hereinabove. Rather the scope of the disclosed technique is defined only by the claims, which follow.

## Claims

1. A weighing scale(s) comprising:
a platform including a plurality of load cells;
an electronics housing, said electronics housing at least including a processor, coupled with said load cells for determining the weight of a load when said load is located on said platform; and
a rotating member rotatably coupling said platform with said electronics housing such that said electronics housing and said platform can rotate one with respect to the other.

2. The weighing scale(s) according to claim 1, wherein said rotating member is one of hinges and a flexible material.

3. The weighing scale(s) according to claim 1, wherein said platform includes a plurality of perforations, wherein each said load cells is located within a respective perforation.

4. The weighing scale(s) according to claim 3, wherein the load cells are located within respective load cell housings which are located in said perforation, and wherein said load cell housings are covered by cell covers.

5. The weighing scale(s) according to claim 1, wherein said electronics housing includes a display for displaying said weight of said load located on said platform.

6. The weighing scale(s) according to claim 1, further including at least one of a body mass index sensor for measuring the body mass index of the load, a fat sensor for measuring the fat of the load, a wireless transceiver and an alarm clock coupled with said processor.

7. The weighing scale(s) according to claim 6, wherein said wireless data transceiver transmits at least one the weight, the body mass index or the fat or any combination thereof of the load.

8. The weighing scale(s) according to claim 6, wherein said alarm clock sounds an alarm at a designated time of the day and stops the alarm when a load is placed on said weighing scale(s).

9. The weighing scale(s) according to claim 1 being covered with a rug.

10. The weighing scale(s) according to claim 1, wherein said rug includes edges exhibiting a declination toward the floor.
